# EUROPEAN PATENT SPECIFICATION

(11) **EP 3 905 973 B1**
(45) Date of publication and mention of the grant of the patent: **15.02.2023**
(21) Application number: 20839062.5
(22) Date of filing: 22.12.2020
(51) Int. Cl.: A61B 17/70, A61B 17/82, A61B 17/84

(54) **BONE REPAIR SYSTEM**
KNOCHENREPARATURSYSTEM
SYSTÈME DE RÉPARATION OSSEUSE

(30) Priority: 17.01.2020 EP 20152526
(43) Date of publication of application: 10.11.2021
(73) Proprietor: Westfälische Wilhelms-Universität Münster, 48100 Münster (DE)
(72) Inventor: SCHULZE, Martin, 48149 Münster (DE); BOCKHOLT, Sebastian, 48161 Münster (DE)
(74) Representative: Ullrich & Naumann PartG mbB
(86) International application number: PCT/EP2020/087649
(87) International publication number: WO 2021/144128

(56) References cited:
- WO-A1-2019/236701
- US-A1- 2010 292 698
- US-A1- 2013 274 748
- US-A1- 2014 277 142
- US-A1- 2018 221 073
- US-B2- 8 740 949
- US-B2- 9 675 386

## Description

The present invention relates to a bone repair system, in particular for treatment of fractures or defects of the pars interarticularis.

A significant number of spine patients suffer from an osteal weakness that can become symptomatic in form of a defect of the pars interarticularis of the lumbar vertebrae, known as spondylolysis. For instance, in Germany, in 2017 more than 15,600 of such cases were diagnosed. Some of these patients can only be helped by surgery. For this purpose, the lytic fractured portion of the vertebral arch is reduced and fixed in position to allow healing of the bone.

Previously applied surgical methods were typically based on the so-called Buck- or Scott procedures, as described, e.g., in Pavlos Panteliadis et al: "Athletic Population with Spondylolysis: Review of Outcomes following Surgical Repair or Conservative Management', in Global Spine Journal 2016;6:615-625. Basically, the Buck procedure is designed as a screw fixation technique with a screw passing through the pars interarticularis to compress the defect. The Scott procedure, on the other hand, utilizes a wire that is passed through the transverse processes of the vertebrae and is tightened around the spinous processes to supplement autologous bone graft repair of the defect by providing compression and stabilization to the defect.

More recent techniques applied for spondylolysis treatment in clinical use are derived from standard screw-rod systems conventionally utilized for the dorsal instrumentation of the spine. Typically, these kinds of systems comprise a variety of small components, including one or more hooks. These hooks (for example, the Harrington Hook) are hitched to the lamina and are then anchored by means of connecting elements onto the screw rods (for reference, see E. Morscher, B. Gerber, J. Fase: "Surgical treatment of spondylolisthesis by bone grafting and direct stabilization of spondylolysis by means of a hook screw", in Archives of Orthopaedic and Traumatic Surgery, September 1984, Volume 103, Issue 3, pp 175-178).

Further alternatives in clinical use are compression screws that are utilized either as a stand-alone system or in combination with autologous bone grafts or different bone substitute materials.

Specifically, document WO 2016/172677 A1 describes an embodiment of a screw system that includes a bone screw, a fastening member, and a securing member. The bone screw includes a threaded shank and a head portion, wherein the head portion has inner walls defining a slot and a recess distal of the slot. The fastening member is received in the recess of the head portion of the bone screw. The securing member has an engaging portion and a set screw rotatably coupled with the engaging portion. The engaging portion is configured to secure the fastening member in the recess of the head portion. The set screw is configured to threadably engage the inner walls of the head portion of bone screw, wherein when the set screw threadably engages the inner walls of the head portion of the bone screw, the engaging portion advances relative to the fastening member to secure the fastening member in the recess of the head portion.

Document US 2018/0132905 A1 discloses a system for fixing a spinal vertebra to a rod. The device comprises a pedicle screw and an anchor lug for fixing the screw to the rod. The device also comprises a flexible strip for connection to the vertebra, a ring secured to the lug, and means secured to the ring, for adjustably blocking the flexible strip in relation to the anchor lug.

Further screw-based systems are described, for instance, in US 8,679,160 B2, US 2011 /0230920 A1 and US 2017/0296239 A1.

Finally, document US 2018/221073 A1 discloses a bone repair system of the type claimed. This system comprises a support member having a bone mounting part configured to be fixed to a first bone or bone fragment so as to mount the support member at the first bone or bone fragment. It further comprises a fastening member including a first and a second end portion and an elongate portion extending between the first and the second end portions, wherein the first end portion of the fastening member is coupled to the support member. The support member comprises a fastening member fixing part, wherein the elongate portion is configured to loop around at least one second bone or bone fragment. The fastening member fixing part of the support member includes fixation means for adjustably securing the second end portion of the fastening member so as to stabilize and fix the first and the second bone or bone fragment with respect to each other. The fixation means include a locking element for receiving the second end portion of the fastening member, wherein the locking element is rotatably mounted with respect to the fastening member fixing part to receive the second end portion of the fasting member.

The prior art spondylolysis treatment systems discussed above are disadvantageous in various aspects. First, it can be recognized that they basically rely on conventional osteosynthetic fracture treatments known, e.g., from the field of oral and maxillofacial surgery or classic traumatology. As such, the systems contain a variety of different (miniaturized) components, including plates, screws, hooks, wire tacks and combinations of the like. However, although the components are adapted in size, they are partly rather bulky and extensive.

In addition, most of the prior art systems entail the assembly of a multitude of individual components. This is not only cumbersome and time-consuming, but also comes along with a restricted variability. Further, each individual component and each connection point between individual components represents a potential source of error. Incorrect couplings between individual components (noticed or unnoticed) may loosen over time or even completely disengage, thereby jeopardizing the result of the treatment. Another disadvantage of the discussed prior art bone repair systems is that the individual components are required to be provided in a variety of different sizes in order to enable sufficient adaptability to individual anatomic conditions.

In view of the above, it is the object of the present invention to improve and further develop a bone repair system in such a way that a quick, easy-to-use and safe implantation is possible, while at the same time sufficient stability is guaranteed.

In accordance with the invention, the aforementioned object is accomplished by a bone repair system comprising the features of claim 1. According to this claim the bone repair system comprises a support member comprising a bone mounting part configured to be fixed to a first bone or bone fragment by means of a bone screw so as to mount the support member at the first bone or bone fragment; and a fastening member including a first and a second end portion and an elongate portion extending between the first and the second end portions, wherein the first end portion of the fastening member is coupled to the support member, wherein the support member comprises a fastening member fixing part, wherein a coupling between the first end portion of the fastening member and the fastening member fixing part is implemented in form of an articulated joint, wherein the elongate portion is configured to loop around at least one second bone or bone fragment, and wherein the fastening member fixing part of the support member includes fixation means for adjustably securing the second end portion of the fastening member so as to stabilize and/or fix the first and the second bone or bone fragment with respect to each other, wherein the fixation means include a locking element for receiving the second end portion of the fastening member, wherein the locking element is rotatably mounted with respect to the fastening member fixing part to receive the second end portion of the fasting member from different angular positions.

According to the invention it has first been recognized that the drawbacks of prior art bone repair systems can be efficiently minimized or even eliminated by strictly reducing the number of individual components that have to be assembled during operative surgery. In this context it can be noted that the bone repair system according to the present invention comprises only a minimum number of components, wherein the support member and the fastening member can even be pre-assembled. Thus, there is no need for assembling individual parts in situ. As a result, the bone repair system according to the present invention has the advantage of easy usage, which in turn comes along with substantial time-saving for the surgeon.

Furthermore, the bone repair system according to the invention has an increased flexibility compared to prior art systems. Due to the fastening member having an elongate portion that is configured to be looped around the concerned bones or bone fragments, the system provides variable anchoring possibilities and can be flexibly adapted to the specific anatomic conditions. In addition, as the fixation means of the support member are configured to allow for an adjustable securing of the fastening member at the support member, the bone repair system proves to be beneficial in terms of enabling secure fixation and/or stabilization of the concerned bone or bone fragments with respect to each other. In particular, the risk of a relaxation or loosening of the fastening member (which may result in insufficient or at least delayed bone healing) is significantly reduced compared to conventional clamping mechanisms.

According to an embodiment the support member of the bone repair system comprises a bone mounting part that is configured for engagement with a bone screw to mount the support member on the first bone or bone fragment. For instance, the bone mounting part may include a substantially ring-shaped portion that is adapted for receiving the head of the bone screw. A conventional pedicel screw with self-tapping threads may be used as bone screw in order to achieve a tight fixation of the support member at the first bone or bone fragment.

According to an embodiment the coupling between the support member and the head of the bone screw is effected by means of an interference fit. In this case, an angulation may be realized between the support member and the head of the bone screw, preferably in the sense of a ball joint mechanism similar to a connecting rod.

Depending on the design, this can be +/- 15 degrees or more. The diameter of the head of the bone screw may be slightly larger than the diameter of the ring-shaped portion of the bone mounting part of the support member. Alternatively, the coupling may be realized by means of a form fit, e.g. based on engaging geometries including a combination of internal/external hexagons, or by means of plastic deformation. In the latter case, suitable material combinations, such as pure titanium and wrought titanium alloy, possibly with a serrated surface, would be required or at least advantageous. According to still another embodiment, coupling between the support member and the head of the bone screw may be effected through mechanical clamping by means of set screw.

With respect to a flexible and secure fixation of the second end portion of the fastening member at the support member, it may be provided that the fixation means are configured to effect securing by means of a form fit. Alternatively, clamping mechanisms or crimping mechanisms, e.g. using a ferrule, or combinations thereof may also be applied.

According to embodiments the elongate portion of the fastening member is formed as a notched or toothed strap or band, as a flexible rack or the like. Such construction accomplishes a two-fold objective. On the one hand, the tooth or the notches of the fastening member may be utilized to interact with corresponding fixation means provided at the support member (e.g., a ratchet mechanism) for adjustably securing the fastening member at the support member. On the other hand, the flexibility of the elongate portion of the fastening member facilitates various adaptations to individual anatomic conditions.

According to embodiments, the support member comprises, in addition to the bone mounting part, a fastening member fixing part. Both parts, i.e. bone mounting part and fastening member fixing part, may be seen as functional elements of the support member. In particular, they may be designed integrally, which means that the support member can be fabricated as a one-piece building component comprising both functional elements.

In an embodiment, the fastening member fixing part of the support member may be composed of two fixing portions: a first fixing portion for anchoring the first end portion of the fastening member, and a second fixing portion including the fixation means for the second end portion of the fastening member. According to a specific embodiment the first fixing portion and the second fixing portion may be arranged substantially one above the other. By means of this construction, mutual interference between both portions can be reduced to a minimum, since the egress of the first end portion of the fastening member from the first fixing portion does not conflict with the ingress of the second portion of the fasting member into the second fixing portion. In fact, egress and ingress are positioned at different levels with respect to the corresponding bone surface, thereby enabling a variety of different angular settings of the fastening member.

With respect to facilitating flexible alignments and adaptations of the fastening member, embodiments of the invention are directed to a bone repair system in which a coupling between the first end portion of the fastening member and the fastening member fixing part of the support member is implemented in form of an articulated joint. For instance, in a specific embodiment the first end portion of the fastening member comprises a spherical head, while the first fixing portion of the fastening member fixing part includes a cavity adapted to accommodate the spherical head. The dimensions of the spherical head on the one hand and of the cavity on the other hand may be chosen such that the fastening member is effectively prevented from being separated from the support member and that at the same time rotations of the spherical head within the cavity are possible, at least to a certain degree, such that the surgeon can appropriately adapt the orientation of the fastening member.

In some embodiments, the fixation means for fastening and/or securing the second end portion of the fastening member at the support member include a locking element that is configured to receive the second end portion of the fasting member preferably from different angular positions. To this end, the support member may be configured to support rotations of the locking element with respect to the fastening member fixing part. For instance, the locking element may be configured as a substantially cylindrically shaped locking element that is rotatably mounted within a corresponding recess formed within the second fixing portion of the fastening member fixing part.

According to an exemplary embodiment, the locking element includes a case having a ratchet mechanism integrated therein, comparable to the mechanism utilized in conventional cable ties. As such, the ratchet mechanism is configured to act on notches or teeth of the fastening member in such a way that the fastening member is prevented from being pulled back, i.e. movement of the fastening member is only possible in a single direction. The case, which is configured for receiving the second end portion of the fastening member through a fastening member insertion opening, may be specifically adapted to the fastening member, in particular in terms of size and the shaping of the fastening member insertion opening, to support easy insertion or threading of the second end portion of the fastening member.

According to embodiments, the second fixing portion of the fastening member fixing part comprises an opening window for insertion of the second end portion of the fastening member into the locking element. With regard to sufficient adaptability and flexibility the opening window may be formed to have a size that facilitates insertion of the second end portion of the fastening member into the locking element under different angles. For instance, the opening window may be formed within an outer wall of the second fixing portion having an opening size that allows for angular alignment of the fastening element in a lateral direction of about at least ± 10°. Depending on the specific area of application, opening windows that allow for angular alignment of the fastening element of up to ± 30° may also be realized.

According to embodiments, the support member and/or the fastening member may be fabricated of any material approved and/or registered as implant material such as, in particular, chrome, molybdenum, or titanium, or an alloy of one or more of these materials, or any composite of implant material, e.g. titanium with polyetheretherketone (PEEK) or carbon fibre reinforced plastic. Alternatively, the support member and/or the fastening member may be fabricated of a resorbable as well as of a non-resorbable material. For instance, different types of polymers may be utilized as resorbable implant material. Alternatively, resolvable light metals may be used which, among other advantages, provide higher strength than the polymers. Furthermore components may be coated with materials improving mechanical conditions or improving biocompatibility, for instance drug eluting substances. For manufacturing the support member and/or the fastening member, different additive manufacturing techniques may be applied, such as laser sintering, LS, selective laser melting, SLM, or electron beam melting, EBM. Generally, in order to facilitate radiologic examinations (e.g., for controlling the healing process), the deployment of materials having X-ray permeability is advantageous.

In an embodiment, the fastening member may include a neck portion that forms the junction between the first end portion and the elongate portion. Since in practical use, particularly this neck portion may be subjected to strong mechanical strain, in particular in form of shear, torsion, and/or tension, the fastening member may be fabricated by integrating a reinforcement within the neck portion.

According to a preferred embodiment, the bone repair system may be deployed for the treatment of spondylolysis, but is not limited to this. As such, the support member may be adapted to be fixed to a human vertebral arch, and the fastening member may be adapted to be looped around the associated pars interarticularis and/or the processus spinosus, the lamina, the processus transversus, and also ribs.

However, as will be easily appreciated by those skilled in the art, the deployment of the bone repair system according to the present invention is not restricted to the specific application mentioned above. In fact, an implantation of the system according to the present invention can be considered in various constellations, as the case may be with minor adaptions of the support member and/or fastening member to the specific anatomic characteristics of the respective bones or bone fragments. For instance, embodiments of the bone repair system may be utilized for stabilizing one or more vertebrae in order to support bone healing.

There are several ways how to design and further develop the teaching of the present invention in an advantageous way. To this end, it is to be referred to the dependent patent claims on the one hand and to the following explanation of preferred examples of embodiments of the invention, illustrated by the drawing on the other hand. In connection with the explanation of the preferred embodiments of the invention by the aid of the drawing, generally preferred embodiments and further developments of the teaching will be explained. In the drawing
- Fig. 1: is a perspective side view illustrating a bone repair system according to an embodiment of the invention,
- Fig. 2a: is a front view of the bone repair system of Fig. 1,
- Fig. 2b: is an enlarged front view of the support member of the bone repair system shown in Fig. 2a,
- Fig. 3a: is a side view of the bone repair system of Fig. 1,
- Fig. 3b: is an enlarged side view of the support member of the bone repair system shown in Fig. 3a,
- Fig. 4: is a perspective side view of a support member of a bone repair system according to an embodiment of the invention,
- Fig. 5: is a perspective side view of a support member of a bone repair system according to another embodiment of the invention,
- Fig. 6: is another perspective side view of the support member of Fig. 5,
- Fig. 7: is a rear view of the support member of Fig. 5,
- Fig. 8: is a first side view of the support member of Fig. 5,
- Fig. 9: is a second side view of the support member of Fig. 5,
- Fig. 10: is a perspective view from above of the locking element of the bone repair system in accordance with an embodiment of the invention,
- Fig. 11: is a perspective view from below of the locking element of Fig. 10,
- Fig. 12: is a side view of the locking element of Fig. 10,
- Fig. 13: is a schematic dorsal view of an implanted bone repair system in accordance with an embodiment of the invention,
- Fig. 14: is another schematic dorsal view of an implanted bone repair system in accordance with an embodiment of the invention,
- Fig. 15: is a schematic lateral view of the implanted bone repair system of Fig. 13,
- Fig. 16: is another schematic lateral view of the implanted bone repair system of Fig. 14, and
- Fig. 17: is schematic lateral view of an implanted bone repair system in accordance with a further embodiment of the invention.

In the following description of embodiments, like reference numeral designate like components.

While a bone repair system according to embodiments of the present invention can be generally utilized in various scenarios where two or more bones or bone fragments have to be fixed and/or stabilized against each other, the embodiments described hereinafter predominantly relate to the treatment of a spondylolysis, which is known as a defect or stress fracture in the pars interarticularis of the human vertebral arch.

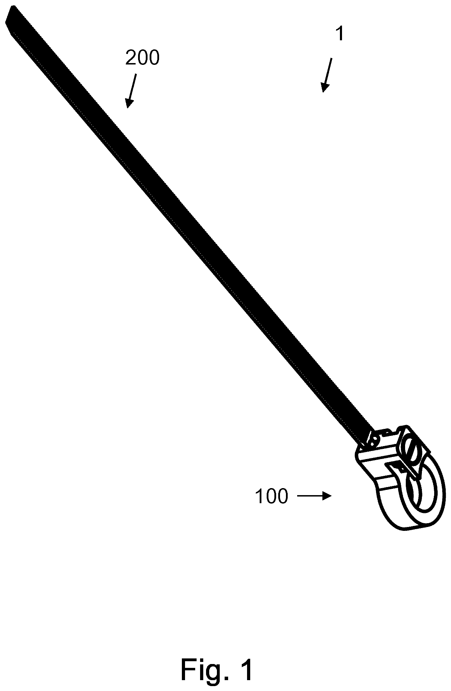

Fig. 1 shows a bone repair system 1 according to an embodiment of the present invention, including a support member 100 and a fastening member 200 coupled to the support member 100. The support member 100 is configured to be fixed to a first bone or bone fragment 400, as will be described in greater detail below. The fastening member 200 is configured to be looped around at least one second bone or bone fragment 410, and to be adjustably secured at the support member 100 so as to stabilize and/or fix the first and the second bone or bone fragment 400, 410 with respect to each other.

As will be appreciated by those skilled in the art, it is one of the major advantages of the bone repair system 1 according to the invention compared to prior art techniques that only a minimum of components has to be handled by the surgeon during surgery. As the support member 100 and the fastening member 200 can be pre-assembled prior to operative surgery, essentially, the operation requires only a single component (together with a bone screw for fastening of the support member 100 at the first bone or bone fragment 400) has to be handled. This means that the actual operative surgery is completely relieved from the requirement of any laborious assembly of individual parts. This advantage comes along with the further advantage of achieving shortened surgery durations, which minimizing the surgical risk and makes the operation less stressful and hazardous for the patient. In addition, the risk of improper assembly or incorrect coupling between individual components is significantly reduced.

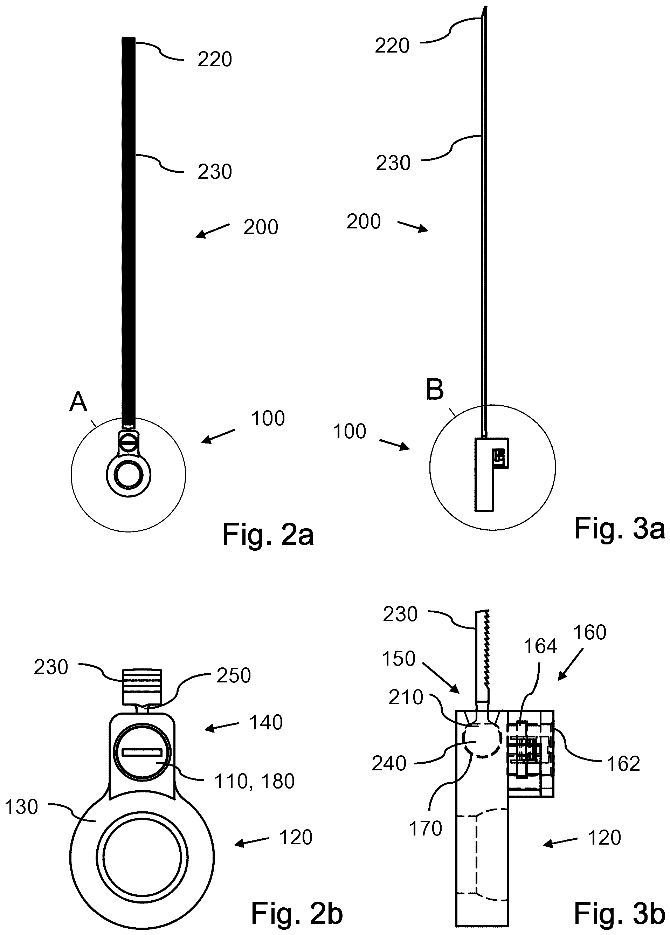

Figs. 2 and 3 show of the embodiment of the bone repair system of Fig. 1 from further perspectives. Figs. 2b and 3b are enlarged views of sections A and B, respectively, of Figs. 2a and 3a. As can be seen, the fastening member 200 comprises a first and a second end portion 210, 220 and an elongate portion 230 extending between the first and the second end portions 210, 220. As can be best obtained from Fig. 3b, the elongate portion 230 is formed as a toothed strap or band. As such, the structure of the fastening member 200 is comparable to that of conventional cable ties. Being formed as a strap or band, the fastening member 200 has sufficient flexibility to adapt to the specific anatomic conditions.

The first end portion 210 of the fastening member 200 is coupled to the support member 100, while the second end portion 220 lies free. After fastening of the support member at 100 a first bone or bone fragment 400 and looping the elongate portion 230 of the fastening member 200 around a second bone or bone fragment 410, the second end portion 220 can be coupled and securely fixed to the support member 100, as will be described in greater detail below.

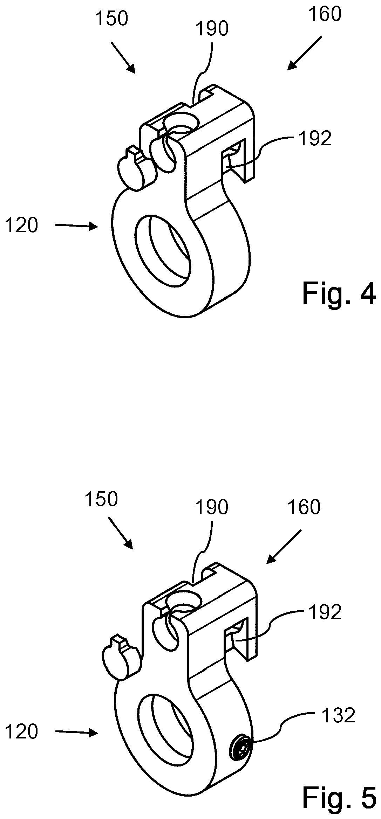

Fig. 4 illustrates the support member 100 of the embodiment of Fig. 1-3 from a perspective view. Essentially, the support member 100 comprises two functional components, namely a bone mounting part 120 and a fastening member fixing part 140. According to preferred embodiments, the support member 100 is formed in one piece, with the bone mounting part 120 and the fastening member fixing part 140 adjoining each other.

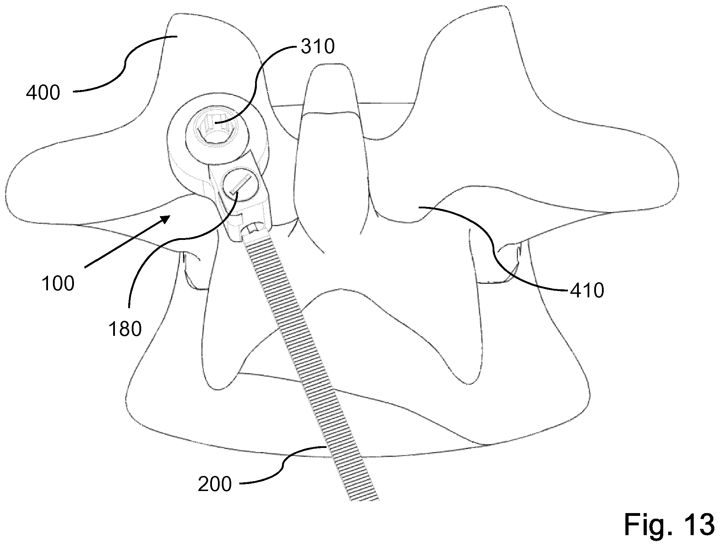

The bone mounting part 120 includes an essentially ring-shaped portion 130 that is configured to receive a bone screw 300 (shown in Figs. 13-16), such as a pedicle screw, for fastening of the support member 100 at the respective first bone or bone fragment 400. In the embodiment shown in Fig. 4, a form fit (or, alternatively, a clamping or crimping mechanism) may be used to effect secure coupling between the bone mounting part 120 and the head 310 of the bone screw 300.

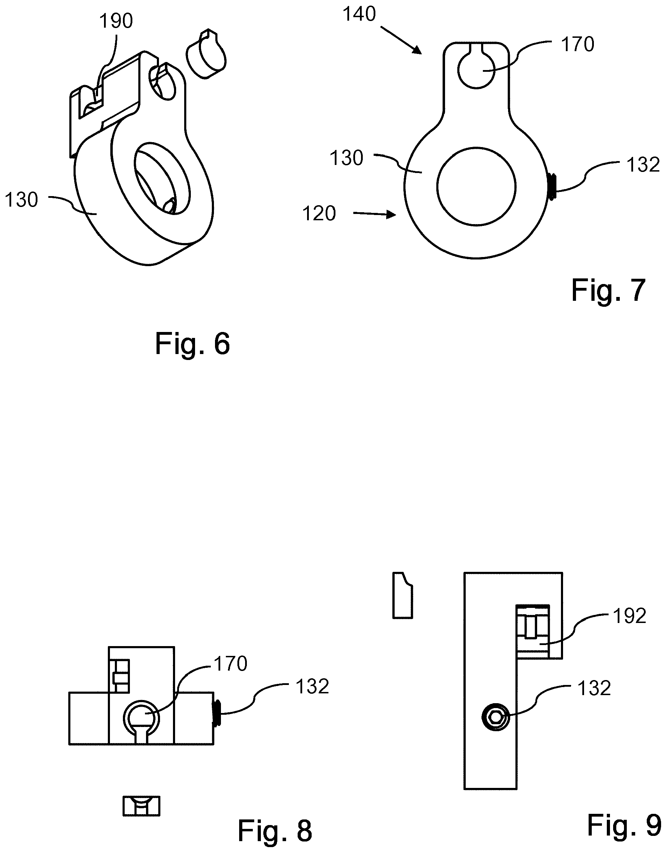

Fig. 5 illustrates a support member 100 according to a similar embodiment. The only difference compared to the embodiment of Fig. 4 is that the coupling between the bone mounting part 120 and the head of the bone screw 300 is realized by a threaded pin 132 provided in a side wall of the essentially ring-shaped portion 130 of the bone mounting part 120. The threaded pin 132 acts as a set screw that exerts a sideward force on the head 310 of the bone screw 300, thereby fixing the bone screw 300 in its desired position. Figs. 6-9 depict the support member 100 of Fig. 5 from further perspectives.

As can be best obtained from Fig. 3b, the fastening member fixing part 140 of the support member 100 includes a first fixing portion 150 for anchoring the first end portion 210 of the fastening member 200, and a second fixing portion 160 for receiving the second end portion 220 of the fastening member 200 after looping the elongate portion 230 around the bone or bone fragments to be stabilized. As seen in a direction perpendicular to the surface of the bone to which the support member 100 is mounted, the first fixing portion 150 and the second fixing portion 160 are substantially arranged one above the other. In this way, any interference between the first and the second end portions 210, 220 of the fastening member 200 is inhibited. Consequently, the direction under which the second end portion 220 can be received within the second fixing portion 160 is completely independent of, i.e. decoupled from the orientation of the first end portion within the first fixing portion 150.

According to the illustrated embodiments, the coupling between the first end portion 210 of the fastening member 200 and the first fixing portion 150 of the support member 100 is realized by means of a ball and socket joint. To this end, the first end portion 210 of the fastening member 200 comprises a spherical head 240 and, correspondingly, the first fixing portion 150 of the fastening member fixing part 140 includes a cavity 170 adapted to accommodate the spherical head 240 of the first end portion 210 of the fastening member 200.

This configuration allows for flexible alignments of the fastening member 200. During surgery, the surgeon may determine the orientation of the fastening member 200 in first instance by proper alignment of the support member 100 itself, i.e. prior to fully tightening the bone screw 300. However, even after having fully tightened the bone screw 300, the ball and socket joint still allows for minor adjustments and corrections of the alignment of the fastening member 200 relative to the involved bones or bone fragments.

As already mentioned above, the fastening member fixing part 140 of the support member 100 includes a second fixing portion 160 for receiving the second end portion 220 of the fastening member 200 after looping the elongate portion 230 around the bone or bone fragments to be stabilized. Specifically, the second fixing portion 160 comprises fixation means 110 for adjustably securing the second end portion 220 of the fastening member 200 so as to stabilize and/or fix the first and the second bone or bone fragment 400, 410 with respect to each other.

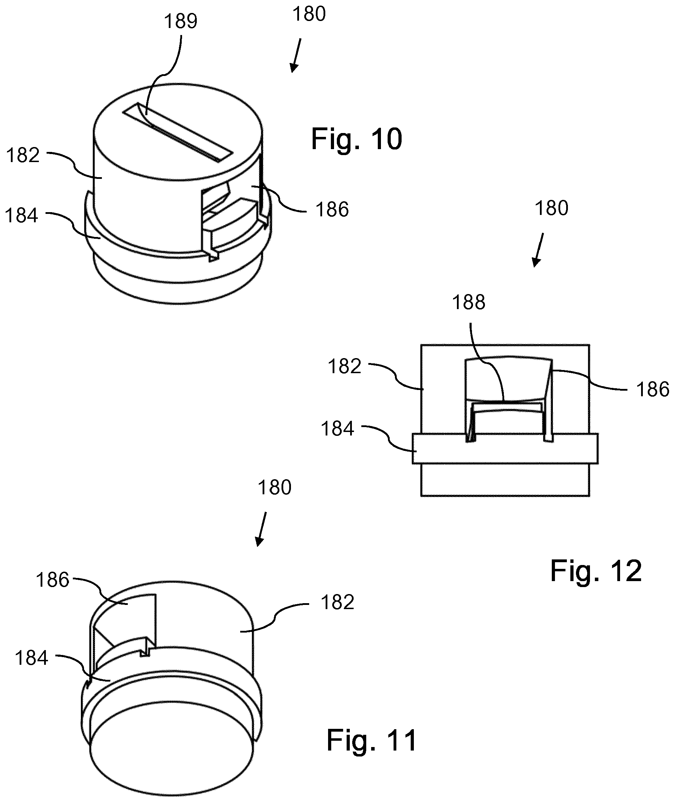

According to the illustrated embodiments, the fixation means 110 include a locking element 180, the locking element 180 having a mainly cylindrical body 182, as shown from different perspectives in Figs. 10-12. Correspondingly, the second fixing portion 160 includes an essentially cylindrical recess 162 in which the locking element 180 is rotatably mounted. Specifically, the recess 162 includes a circumferential groove 164 formed for engagement with a respective bulge portion 184 of the cylindrical body 182 of the locking element 180, so as to prevent the locking element 180 from getting disengaged from the support member 100.

As can be obtained from Figs. 10-12, the locking element 180 comprises a case 186 including a first opening window that is adapted for receiving the second end portion 220 of the fastening member 200, and a second opening window, disposed on the opposite side of the first opening window, for leading the fastening member 200 out of the locking element 180. A ratchet mechanism 188 is integrated within the case 186, wherein the ratchet mechanism 188 is configured to act on the notches or teeth of the fastening member 200 so as to prevent the fastening member 200 from being pulled back. In other words, once the second end portion 220 of the fastening member 200 has been pulled through the case 186 and past the ratchet 188, it is prevented from being pulled back, i.e. the resulting loop may only be pulled tighter.

As can be best seen in Figs. 4, 5 and 9, the second fixing portion 160 of the fastening member fixing part 140 comprises an ingress opening 190 for receiving the second end portion 220 of the fastening member 200 within the second fixing portion 160 and for its insertion into the locking element 180. Likewise, the second fixing portion 160 comprises an egress opening 192 for leading the second end portion 220 of the fastening member 200 out of the second fixing portion 160. For instance, the openings 190, 192 may be formed as recesses within an outer wall of the second fixing portion 160. According to embodiments, both openings 190, 192 may have an opening size sufficiently large that angular alignment of the fastening element in a lateral direction of about at least ± 10° can be accomplished. As already mentioned above and as appreciated by those skilled in the art, depending on the anatomic conditions, opening windows with larger opening angels (e.g. ± 30°) or with an asymmetry between ingress opening 190 and egress opening 192 may be realized. In order to bring the opening windows of the case 186 of the locking element 180 into alignment with the openings 190, 192 of the second fixing portion 160, a slit 188 is formed in the upper surface of the locking element 180. The slit 188 (or, according to alternative embodiments, a cross slot, a hexagon socket, an internal torx/stardrive, or the like) is adapted for engagement with a respective tool by which the surgeon can easily effect appropriate rotation of the locking element 180.

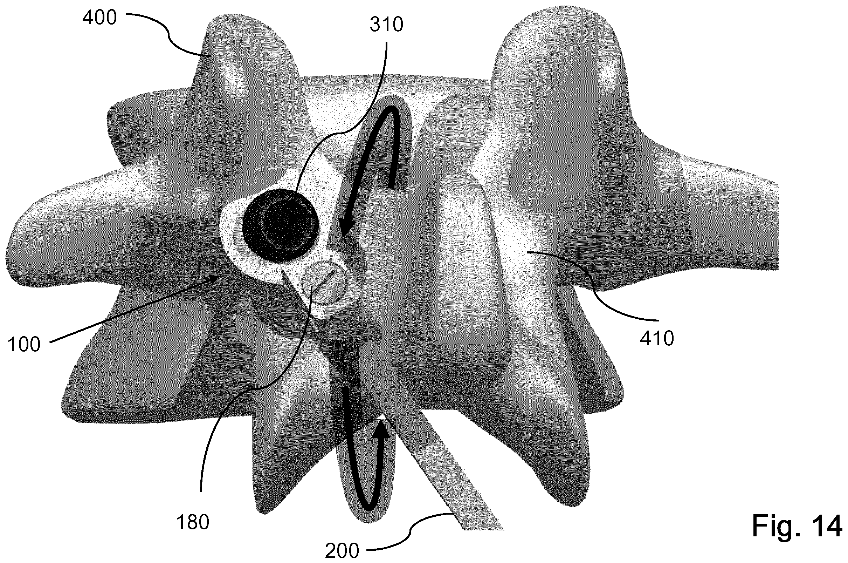

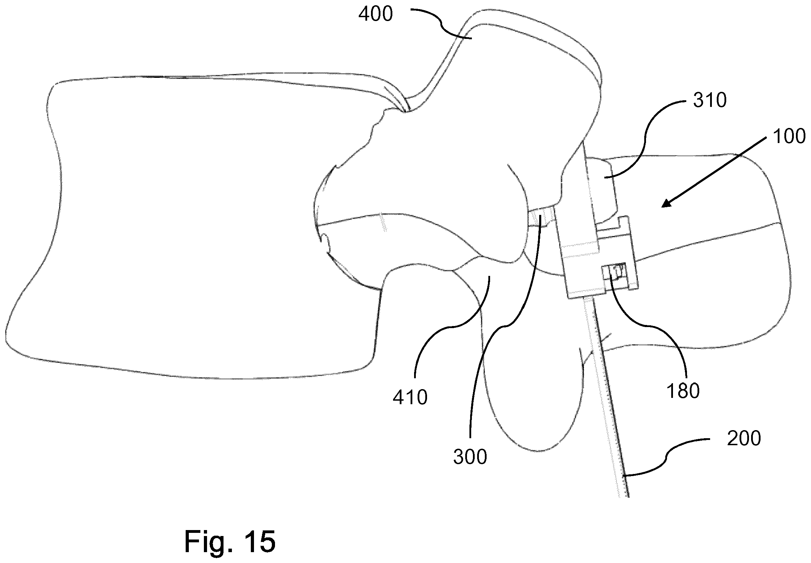

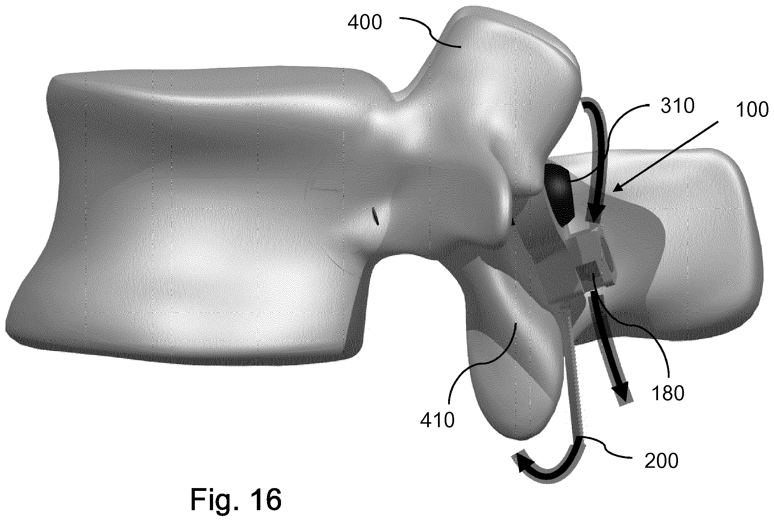

Figs. 13-16 schematically depict an implanted bone repair system in the specific case of treatment of a fracture and/or defect of the pars interarticularis vertebrae (lytic, traumatic, degenerative, due to growth, etc.) in accordance with embodiments of the invention. Figs. 13 and 15 illustrate the implant in dorsal views, while Figs. 14 and 16 show the respective lateral views. In this case, the support member 10 is mounted to the affected vertebral arch as the first bone or bone fragment 400, and the fastening member 200 is looped around the associated pars interarticularis as the second bone or bone fragment 410.

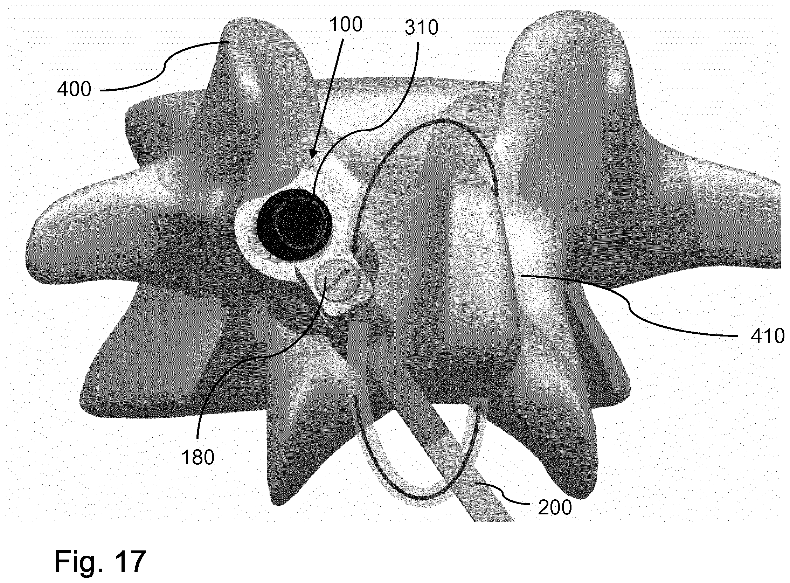

Fig. 17 depicts a different implant situation. While the support member 10 is still mounted to the vertebral arch as the first bone or bone fragment 400, the fastening member 200 is not looped around the associated pars interarticularis but, instead, around the associated processus spinosus as the second bone or bone fragment 410.

According to still further embodiments, not shown in the drawings, the fastening member 200 may be looped around the lamina, the processus transversus, and also around a rib as the second bone or bone fragment 410.

By the implantation of a bone repair system in accordance with embodiments of the present invention, one or more of the following advantages can be achieved:
- time saving for the surgeon through easy application;
- secure fixation of the involved bone fragments by looping with the fastening member;
- minimization of loosening, which could lead to protracted or failing bone healing;
- reduction of the risk of misplacement and the risk of injury of nerve roots, spinal cord and spinal membrane;
- cost-effective manufacturing.

### List of reference numbers

- 1: bone repair system
- 100: support member
- 110: fixation means
- 120: bone mounting part
- 130: ring-shaped portion
- 132: threaded pin
- 140: fastening member fixing part
- 150: first fixing portion
- 160: second fixing portion
- 162: cylindrical recess
- 164: circumferential groove
- 170: cavity
- 180: locking element
- 182: cylindrical body
- 184: bulge portion
- 186: case
- 188: ratchet mechanism
- 189: slit
- 190: opening window (ingress opening)
- 192: opening window (egress opening)
- 200: fastening member
- 210: first end portion
- 220: second end portion
- 230: elongate portion
- 240: spherical head
- 250: neck portion
- 300: bone screw
- 310: screw head
- 400: first bone or bone fragment
- 410: second bone or bone fragment

## Claims

1. A bone repair system, the system comprising:
a support member (100) comprising a bone mounting part (120) configured to be fixed to a first bone or bone fragment (400) by means of a bone screw so as to mount the support member at the first bone or bone fragment, and
a fastening member (200) including a first and a second end portion (210, 220) and an elongate portion (230) extending between the first and the second end portions (210, 220),
wherein the first end portion (210) of the fastening member (200) is coupled to the support member (100), wherein the support member (100) comprises a fastening member fixing part (140), wherein a coupling between the first end portion (210) of the fastening member (200) and the fastening member fixing part (140) is implemented in form of an articulated joint,
wherein the elongate portion (230) is configured to loop around at least one second bone or bone fragment (410), and
wherein the fastening member fixing part (140) of the support member (100) includes fixation means (110) for adjustably securing the second end portion (220) of the fastening member (200) so as to stabilize and/or fix the first and the second bone or bone fragment (400; 410) with respect to each other, wherein the fixation means (110) include a locking element (180) for receiving the second end portion (220) of the fastening member (200), wherein the locking element (180) is rotatably mounted with respect to the fastening member fixing part (140) to receive the second end portion (220) of the fasting member (200) from different angular positions.

2. The bone repair system according to claim 1, wherein the bone mounting part (120) of the support member (100) includes a substantially ring-shaped portion (130) adapted for receiving the head (310) of a bone screw (300), in particular a pedicel screw.

3. The bone repair system according to claim 1 or 2, wherein a coupling between the support member (100) and the head (310) of a bone screw (300) is effected by means of an interference fit, by means of a form fit or by means of plastic deformation.

4. The bone repair system according to any of claim 1 to 3, wherein the fixation means (110) are configured to effect securing of the second end portion (220) of the fastening member (200) by means of a form fit, by means of a clamping mechanism or by means of a crimping mechanism.

5. The bone repair system according to any of claims 1 to 4, wherein the elongate portion (230) of the fastening member (200) is formed as a notched or toothed strap, band or flexible rack.

6. The bone repair system according to any of claims 1 to 5, wherein the support member (100) comprises a fastening member fixing part (140) including
a first fixing portion (150) for anchoring the first end portion (210) of the fastening member (200), and
a second fixing portion (160) including the fixation means (110) for the second end portion (220) of the fastening member (200).

7. The bone repair system according to claim 6, wherein the first fixing portion (150) and the second fixing portion (160) are substantially arranged one above the other.

8. The bone repair system according to claim 6 or 7, wherein the first end portion (210) of the fastening member (200) comprises a spherical head (240), and wherein the first fixing portion (150) of the fastening member fixing part (140) includes a cavity (170) adapted to accommodate the spherical head (240) of the first end portion (210) of the fastening member (200).

9. The bone repair system according to any of claims 6 to 8, wherein the locking element (180) of the fixation means (110) is shaped substantially cylindrically, wherein the locking element (180) is rotatably mounted within a corresponding recess formed within the second fixing portion (160) of the fastening member fixing part (140).

10. The bone repair system according to claim 9, wherein the locking element (180) includes a case having a ratchet mechanism integrated therein,
wherein the case is adapted for receiving the second end portion (220) of the fastening member (200), and
wherein the ratchet mechanism is configured to act on notches or teeth of the fastening member (200) so as to prevent the fastening member (200) from being pulled back.

11. The bone repair system according to claim 9 or 10, wherein the second fixing portion (160) of the fastening member fixing part (140) comprises an opening window (190), the opening window (190) being adapted to allow insertion of the second end portion (220) of the fastening member (200) into the locking element (180) under different angles.

12. The bone repair system according to any of claims 1 to 11, wherein the fastening member (200) is fabricated of chrome, molybdenum, or titanium, or of an alloy thereof, or of a composite, in particular titanium with polyetheretherketone, PEEK, or a carbon fibre reinforced plastic.

13. The bone repair system according to any of claims 1 to 12, wherein at least one of the support member (100) and the fastening member (200) is fabricated of a resorbable material.

14. The bone repair system according to any of claims 1 to 13, wherein the fastening member (140) includes a reinforcement within a neck portion (250) adjoining the first end portion (210).

15. The bone repair system according to any of claims 1 to 14, wherein the support member (100) is adapted to be fixed to a human vertebral arch, and wherein the fastening member (200) is adapted to be looped around the associated pars interarticularis, the associated processus spinosus, the lamina, the processus transversus, or a rib for treatment of a spondylolysis.

## Patentansprüche

1. Knochenreparatursystem, wobei das System umfasst:
ein Support-Element (100) mit einem Knochenbefestigungsteil (120), das so konfiguriert ist, dass es mittels einer Knochenschraube an einem ersten Knochen oder Knochenfragment (400) befestigt werden kann, um das Stützelement an dem ersten Knochen oder Knochenfragment zu montieren, und
ein Befestigungselement (200) mit einem ersten und einem zweiten Endabschnitt (210, 220) und einem länglichen Abschnitt (230), der sich zwischen dem ersten und dem zweiten Endabschnitt (210, 220) erstreckt,
wobei der erste Endabschnitt (210) des Befestigungselements (200) mit dem Support-Element (100) gekoppelt ist, wobei das Support-Element (100) ein Befestigungselement-Fixierungsteil (140) aufweist, wobei eine Kopplung zwischen dem ersten Endabschnitt (210) des Befestigungselements (200) und dem Befestigungselement-Fixierungsteil (140) in Form einer gelenkigen Verbindung ausgeführt ist,
wobei der längliche Abschnitt (230) zum Umschlingen mindestens eines zweiten Knochens oder Knochenfragments (410) ausgebildet ist, und
wobei das Befestigungselement-Fixierungsteil (140) des Support-Elements (100) Fixierungsmittel (110) zum einstellbaren Befestigen des zweiten Endabschnitts (220) des Befestigungselements (200) umfasst, um den ersten und den zweiten Knochen oder das erste und das zweite Knochenfragment (400; 410) in Bezug zueinander zu stabilisieren und/oder zu fixieren, wobei die Fixierungsmittel (110) ein Verriegelungselement (180) zur Aufnahme des zweiten Endabschnitts (220) des Befestigungselements (200) umfassen, wobei das Verriegelungselement (180) in Bezug auf das Befestigungselement-Fixierungsteil (140) drehbar montiert ist, um den zweiten Endabschnitt (220) des Befestigungselements (200) aus verschiedenen Winkelpositionen aufzunehmen.

2. Knochenreparatursystem nach Anspruch 1, wobei das Knochenbefestigungsteil (120) des Support-Elements (100) einen im Wesentlichen ringförmigen Abschnitt (130) aufweist, der zur Aufnahme des Kopfes (310) einer Knochenschraube (300), insbesondere einer Pedikelschraube, geeignet ist.

3. Knochenreparatursystem nach Anspruch 1 oder 2, wobei eine Kopplung zwischen dem Support-Element (100) und dem Kopf (310) einer Knochenschraube (300) mittels eines Presssitzes, mittels eines Formschlusses oder mittels plastischer Verformung bewirkt wird.

4. Knochenreparatursystem nach einem der Ansprüche 1 bis 3, wobei die Fixierungsmittel (110) so konfiguriert sind, dass sie die Befestigung des zweiten Endabschnitts (220) des Befestigungselements (200) mittels eines Formschlusses, mittels eines Klemmmechanismus oder mittels eines Quetschmechanismus bewirken.

5. Knochenreparatursystem nach einem der Ansprüche 1 bis 4, wobei der längliche Abschnitt (230) des Befestigungselements (200) als gekerbte(s/r) oder gezahnte(s/r) Riemen, Band oder flexible Zahnstange ausgebildet ist.

6. Knochenreparatursystem nach einem der Ansprüche 1 bis 5, wobei das Support-Element (100) ein Befestigungselement-Fixierungsteil (140) umfasst, das Folgendes umfasst:
einen ersten Fixierungsabschnitt (150) zur Verankerung des ersten Endabschnitts (210) des Befestigungselements (200), und
einen zweiten Fixierungsabschnitt (160), der die Fixierungsmittel (110) für den zweiten Endabschnitt (220) des Befestigungselements (200) umfasst.

7. Knochenreparatursystem nach Anspruch 6, wobei der erste Fixierungsabschnitt (150) und der zweite Fixierungsabschnitt (160) im Wesentlichen übereinander angeordnet sind.

8. Knochenreparatursystem nach Anspruch 6 oder 7, wobei der erste Endabschnitt (210) des Befestigungselements (200) einen kugelförmigen Kopf (240) aufweist, und wobei der erste Fixierungsabschnitt (150) des Befestigungselement-Fixierungsteils (140) einen Hohlraum (170) aufweist, der angepasst ist, den kugelförmigen Kopf (240) des ersten Endabschnitts (210) des Befestigungselements (200) aufzunehmen.

9. Knochenreparatursystem nach einem der Ansprüche 6 bis 8, wobei das Verriegelungselement (180) des Fixierungsmittels (110) im Wesentlichen zylindrisch geformt ist, wobei das Verriegelungselement (180) drehbar in einer entsprechenden Aussparung montiert ist, die in dem zweiten Fixierungsabschnitt (160) des Befestigungselement-Fixierungsteils (140) ausgebildet ist.

10. Knochenreparatursystem nach Anspruch 9, wobei das Verriegelungselement (180) ein Gehäuse mit einem darin integrierten Ratschenmechanismus aufweist,
wobei das Gehäuse zur Aufnahme des zweiten Endabschnitts (220) des Befestigungselements (200) geeignet ist, und
wobei der Ratschenmechanismus so konfiguriert ist, dass er auf Kerben oder Zähne des Befestigungselements (200) wirkt, um zu verhindern, dass das Befestigungselement (200) zurückgezogen wird.

11. Knochenreparatursystem nach Anspruch 9 oder 10, wobei der zweite Fixierungsabschnitt (160) des Befestigungselement-Fixierungsteils (140) ein Öffnungsfenster (190) aufweist, wobei das Öffnungsfenster (190) geeignet ist, das Einführen des zweiten Endabschnitts (220) des Befestigungselements (200) in das Verriegelungselement (180) unter verschiedenen Winkeln zu ermöglichen.

12. Knochenreparatursystem nach einem der Ansprüche 1 bis 11, wobei das Befestigungselement (200) aus Chrom, Molybdän oder Titan oder aus einer Legierung davon oder aus einem Verbundwerkstoff, insbesondere Titan mit Polyetheretherketon, PEEK, oder einem kohlenstofffaserverstärkten Kunststoff, hergestellt ist.

13. Knochenreparatursystem nach einem der Ansprüche 1 bis 12, bei dem das Supportelement (100) und/oder das Befestigungselement (200) aus einem resorbierbaren Material hergestellt sind.

14. Das Knochenreparatursystem nach einem der Ansprüche 1 bis 13, wobei das Befestigungselement (140) eine Verstärkung in einem an den ersten Endabschnitt (210) angrenzenden Halsabschnitt (250) aufweist.

15. Knochenreparatursystem nach einem der Ansprüche 1 bis 14, wobei das Support-Element (100) so angepasst ist, dass es an einem menschlichen Wirbelbogen befestigt werden kann, und wobei das Befestigungselement (200) so angepasst ist, dass es um die zugehörige Pars interarticularis, den zugehörigen Processus spinosus, die Lamina, den Processus transversus oder eine Rippe zur Behandlung einer Spondylolyse geschlungen werden kann.

## Revendications

1. Système de réparation osseuse, le système comprenant :
un élément de support (100) comprenant une partie de montage d'os (120) configurée pour être fixée à un premier os ou fragment osseux (400) au moyen d'une vis osseuse afin de monter l'élément de support sur le premier os ou fragment osseux, et
un élément de fixation (200) comprenant une première et une seconde partie d'extrémité (210, 220) et une partie allongée (230) s'étendant entre la première et la seconde partie d'extrémité (210, 220),
dans lequel la première partie d'extrémité (210) de l'élément de fixation (200) est couplée à l'élément de support (100), dans lequel l'élément de support (100) comprend une partie de fixation d'élément de fixation (140), dans lequel un couplage entre la première partie d'extrémité (210) de l'élément de fixation (200) et la partie de fixation d'élément de fixation (140) est mis en oeuvre sous la forme d'un joint articulé,
dans lequel la partie allongée (230) est configurée pour tourner autour d'au moins un second os ou fragment osseux (410), et
dans lequel la partie de fixation d'élément de fixation (140) de l'élément de support (100) comprend des moyens de fixation (110) pour fixer, de manière ajustable, la seconde partie d'extrémité (220) de l'élément de fixation (200) pour stabiliser et/ou fixer le premier et le second os ou fragment osseux (400 ; 410) l'un par rapport à l'autre, dans lequel les moyens de fixation (110) comprennent un élément de verrouillage (180) pour recevoir la seconde partie d'extrémité (220) de l'élément de fixation (200), dans lequel l'élément de verrouillage (180) est monté, en rotation, par rapport à la partie de fixation d'élément de fixation (140) pour recevoir la seconde partie d'extrémité (220) de l'élément de fixation (200) depuis différentes positions angulaires.

2. Système de réparation osseuse selon la revendication 1, dans lequel la partie de montage d'os (120) de l'élément de support (100) comprend une partie de forme sensiblement annulaire (130) adaptée pour recevoir la tête (310) d'une vis osseuse (300), en particulier une vis pédiculaire.

3. Système de réparation osseuse selon la revendication 1 ou 2, dans lequel un couplage entre l'élément de support (100) et la tête (310) d'une vis osseuse (300) est effectué au moyen d'un ajustement avec serrage, au moyen d'un ajustement par complémentarité de formes ou au moyen de déformation plastique.

4. Système de réparation osseuse selon l'une quelconque des revendications 1 à 3, dans lequel les moyens de fixation (110) sont configurés pour effectuer la fixation de la seconde partie d'extrémité (220) de l'élément de fixation (200) au moyen d'un ajustement par complémentarité de formes, au moyen d'un mécanisme de serrage ou au moyen d'un mécanisme de sertissage.

5. Système de réparation osseuse selon l'une quelconque des revendications 1 à 4, dans lequel la partie allongée (230) de l'élément de fixation (200) est formée comme une sangle crantée ou dentée, une bande ou une crémaillère souple.

6. Système de réparation osseuse selon l'une quelconque des revendications 1 à 5, dans lequel l'élément de support (100) comprend une partie de fixation d'élément de fixation (140) comprenant :
une première partie de fixation (150) pour ancrer la première partie d'extrémité (210) de l'élément de fixation (200), et
une seconde partie de fixation (160) comprenant les moyens de fixation (110) pour la seconde partie d'extrémité (220) de l'élément de fixation (200).

7. Système de réparation osseuse selon la revendication 6, dans lequel la première partie de fixation (150) et la seconde partie de fixation (160) sont agencées sensiblement l'une au-dessus de l'autre.

8. Système de réparation osseuse selon la revendication 6 ou 7, dans lequel la première partie d'extrémité (210) de l'élément de fixation (200) comprend une tête sphérique (240), et dans lequel la première partie de fixation (150) de la partie de fixation d'élément de fixation (140) comprend une cavité (170) adaptée pour accueillir la tête sphérique (240) de la première partie d'extrémité (210) de l'élément de fixation (200).

9. Système de réparation osseuse selon l'une quelconque des revendications 6 à 8, dans lequel l'élément de verrouillage (180) des moyens de fixation (110) est formé de manière sensiblement cylindrique, dans lequel l'élément de verrouillage (180) est monté, en rotation, dans un évidement correspondant formé à l'intérieur de la seconde partie de fixation (160) de la partie de fixation d'élément de fixation (140).

10. Système de réparation osseuse selon la revendication 9, dans lequel l'élément de verrouillage (180) comprend un boîtier ayant un mécanisme à cliquet intégré à l'intérieur de ce dernier,
dans lequel le boîtier est adapté pour recevoir la seconde partie d'extrémité (220) de l'élément de fixation (200), et
dans lequel le mécanisme à cliquet est configuré pour agir sur les encoches ou les dents de l'élément de fixation (200) afin d'empêcher l'élément de fixation (200) d'être tiré en arrière.

11. Système de réparation osseuse selon la revendication 9 ou 10, dans lequel la seconde partie de fixation (160) de la partie de fixation d'élément de fixation (140) comprend une fenêtre d'ouverture (190), la fenêtre d'ouverture (190) étant adaptée pour permettre l'insertion de la seconde partie d'extrémité (220) de l'élément de fixation (200) dans l'élément de verrouillage (180) sous différents angles.

12. Système de réparation osseuse selon l'une quelconque des revendications 1 à 11, dans lequel l'élément de fixation (200) est fabriqué à partir de chrome, de molybdène ou de titane ou avec leur alliage, ou avec un composite, en particulier du titane avec du polyétheréthercétone, PEEK ou du plastique renforcé en fibres de carbone.

13. Système de réparation osseuse selon l'une quelconque des revendications 1 à 12, dans lequel au moins l'un parmi l'élément de support (100) et l'élément de fixation (200) est fabriqué avec un matériau résorbable.

14. Système de réparation osseuse selon l'une quelconque des revendications 1 à 13, dans lequel l'élément de fixation (140) comprend un renforcement dans une partie de col (250) attenante à la première partie d'extrémité (210).

15. Système de réparation osseuse selon l'une quelconque des revendications 1 à 14, dans lequel l'élément de support (100) est adapté pour être fixé à un arc vertébral humain, et dans lequel l'élément de fixation (200) est adapté pour être entraîné en rotation autour des isthmes vertébraux associés, de l'apophyse épineuse associée, la lamina, l'apophyse transverse ou une côte pour le traitement d'une spondylolyse.
